# EUROPEAN PATENT APPLICATION

(11) **EP 2 965 731 A1**
(43) Date of publication of application: **13.01.2016**
(21) Application number: 15182175.8
(22) Date of filing: 28.02.2011
(51) Int. Cl.: A61F 13/38, G01N 1/02, A61F 15/00, A61B 10/02, A61B 10/00, B01L 3/00

(54) **EVIDENCE COLLECTOR WITH INTEGRAL QUANTIFIED REAGENTS AND METHOD OF MODULATING SPECIMEN DRYING TIME**

(30) Priority: 27.02.2010 US 714477; 24.02.2011 US 201113034541; 25.02.2011 US 201113035577
(62) Divisional of application: 11748238.0
(71) Applicant: The Bode Technology Group, Inc, Lorton, VA 22079 (US)
(72) Inventor: Sangha, Jangbir S., Overland Park, KS Kansas 66209 (US)
(74) Representative: Teipel, Stephan

(57) **Abstract**

Apparatus and methods are provided for evidence specimen collection having integral reagent holders to hold reagent vials and having drying agent or desiccant holding areas that permit the renewal of the desiccant and permit the introduction of variously size desiccant qualities to allow modulation of the specimen drying time to achieve early stabilization of specimens while holding the specimen in an isolated drying area during storage and shipment and for simultaneous collection of multiple evidence samples with simultaneous storage, drying, marking, evidence security and shipping provided and with the provision for simultaneous storage, drying, marking, evidence security and shipping provided for a control specimen.

## Description

### Cross Reference to Related Application

This International application claims priority under 35 U.S.C. 119(e) and under 35 U.S.C. 120 to U.S. Application 13/035,577 filed February 25, 2011, U.S. Application 13/034,541 filed February 24, 2011, U.S. Application 12/714,477 filed February 27, 2010 and United States Patent Application Serial No. 11/653,116 filed January 12, 2007 based upon copending U.S. Provisional Application Serial No. 60/758,855 filed January 13, 2006 and U.S. Application 11/787,313 filed April 16, 2007 based upon copending U.S. Provisional Application 60/792,057 filed April 114, 2006 and copending U.S. Provisional Application Serial No. 61/172,771 filed April 25, 2009, based upon copending U.S. Application 11/699,807 filed January 30, 2007 based upon copending U.S. Provisional Application 60/815,801 filed June 22, 2006 all of which are incorporated herein by reference.

### Field of the Invention

The field of the invention is directed to apparatus and methods for field collection and transport and analysis of laboratory specimens and crime scene evidence samples. The field of the invention also relates to a method of modulating the drying time of such specimens or evidence samples after collection to achieve rapid drying of the specimens or evidence samples based on the quantity of specimen and the quantity of moisture present in the specimens or evidence samples.

### Background of the Invention

The present embodiments provide a specimen collection and drying and transport and storage device that can be used for laboratory and forensic purposes to gather samples and/or specimens and to then dry the sample and/or specimen during transport and/or storage prior to testing of the sample or specimen. All this can be accomplished in the present embodiments while providing assurance that the chain of custody has been preserved and that the collected specimen or sample has not been switched during the changing of the drying agent employed to dry the specimen.

More particularly, the embodiments relate to a specimen collection apparatus for collecting such samples and stabilizing the specimens and preserving them from contamination prior to laboratory analysis. Therefore, an apparatus is provided in which the specimen collector is enclosed after collection of the sample thereon to protect the sample from contamination. The embodiments also allow exposure of the specimen or evidence sample to a drying agent to dry and stabilize the specimen to promote specimen integrity by providing rapid drying soon after specimen collection. Further, the embodiments allow the user to renew, or change-out, exhausted drying agent without disturbing the specimen. And, the embodiments allow the user to select and insert variously sized desiccant packets to modulate the drying time of the collected specimen or sample depending upon user desires for the particular specimen or sample.

In one embodiment simultaneous, identical, dual specimen or sample collection is provided which allows two identical specimens to be simultaneously collected in one motion by the user and to then simultaneously deliver the dual and identical specimens to a single housing to thereby assure that the specimen or evidence samples receive simultaneous and identical protection, drying conditions and transport conditions. Further, the embodiment allows one of the two identical and simultaneously collected specimens to remain untouched or unused and to be archived without removal of the specimen from the original housing into which it was inserted after collection. This may be accomplished while allowing the other of the two identical and simultaneously collected specimens to be removed from the housing or for a portion thereof removed for testing.

Crime scene evidence is collected to establish facts related to a crime or a suspected crime and for identification and/or elimination of suspects and may be presented at a trial for the determination of guilt or innocence of accused individuals. Often, the evidence includes objects, documents, fingerprints, photographs of the scene, and the like. Additionally, the evidence may include unknown substances or substances with a suspected identity, where the identity needs to be determined or confirmed. Such substances may be very small in quantity, may be dispersed over a comparatively large area, and may include materials such as: body fluids, hairs, flakes of skin such as skin cells, fibers, drugs, various chemicals, gunpowder residue, flammable materials, tobacco ashes, cosmetics, and the like. Such materials may be collected at a scene and subjected to chemical and/or DNA analysis for identification or for association with a particular individual.

Currently, for collecting specimen samples, investigators typically use fibrous swabs, such as swabs made of fibers of cotton, cellulose, rayon, polyester, polyester foam and other types of fibers. Such swabs not only absorb liquids and solids suspended in liquids but also trap dry substances such as particulate materials. Prior to use, the swabs are kept in closed sterile bags or containers to maintain sterility. After specimen collection the swabs and are placed into a similar bag or container to avoid contamination of the sample gathered during transportation. Once the swab is placed in a container after specimen collection, the container is usually marked with a time, the date, the identity of the investigator and other information to establish a chain of custody of the sample.

Conventional swabs are formed of a "stick" such as a shaft of wood, tubular plastic, or tubular or rolled paper with a pad of cotton or other fiber, sponge material, or other absorbent material attached to the end of the shaft, either mechanically or by an inert adhesive. A problem with conventional swabs is that there is a danger of contamination of the sample if it is necessary to put the swab down, for example, to open a bag or container in which the swab will be placed. Also, if it is necessary to set the swab down to dry, in a propped up condition or extending over the edge of a table, there is a risk of contamination of the sample.

The present embodiments provide an apparatus and method for collecting solid, fluid or particulate evidence specimens related to any type of situation in which evidence collection is required. Such evidence collection can be associated with crime scenes or can simply be the collection of a DNA sample from a human being in the course of a traffic stop or a paternity investigation. Suitable specimens for collection using the present devices are, in general, that evidence which is located on a surface or on a human being and which can be physically contacted by an evidence collection device to thereby obtain a sample of the evidence. Examples of such evidence specimens might be any type of biological fluid, either wet or dried, such as blood, urine or saliva, or any unknown substance which is visible or invisible and which can be located allowing for collection of a specimen of the evidence and capture of such a sample on a specimen collector of the type described hereinafter. As previously mentioned, it will be appreciated that such specimen collection devices are widely used in criminal investigations, but also are used increasingly in traffic stop situations or traffic arrest situations in which it is desirable to obtain a DNA sample from the suspect as part of a criminal records database requirement.

Therefore, for proper evidence collection that can be used in court to support a conviction, it is necessary that investigators have at their disposal a device and method of collection that dries the collected specimen shortly after collection to promote sample integrity by stabilizing the specimen by drying. It is additionally important that the apparatus promotes accuracy of specimen collection and reproducibility of specimen collection and protection of specimens from contamination while providing a device that enables a verifiable chain of custody while allowing continuous renewal of drying agents positioned adjacent to the specimen and while providing quantified specimen dilution during collection procedures and all without contributing to contamination of the crime scene by introducing extraneous material into the crime scene.

### SUMMARY OF THE INVENTION

A first embodiment provides a specimen collector and container which may be used to collect a specimen with the container operating as a handle for the manipulation of the specimen collector and then subsequently the container may be used to receive the specimen collector therein for drying of the specimen within the container and for shipping of the specimen in a protected manner to an evidence room or to a laboratory and while a drying agent in the container, capable of being renewed without disturbing the specimen, speeds the drying of the collected specimen.

In another embodiment, the present device provides a specimen collector and container having all the above features and further providing the crime scene investigator with interchangeable, quantified specimen collection reagents and variable specimen collection reagents, which due to the device structure are fully and accurately absorbable by the specimen collection swab.

In another embodiment, the collection device provides for a swab on a specimen collector which swab can be conveniently detached from the specimen collector and specifically from the shaft connecting the swab to the specimen collector through use of a coaxially mounted tube which surrounds the shaft on which the swab is mounted. The coaxially mounted tube is provided with a terminal end which is located proximate to an area on the shaft where it is desired to have a point of breakage, or break-point location on the shaft, to separate the swab from the shaft to allow the swab to be separated from the specimen collector and to allow the swab to be deposited within a separate container. Another embodiment allow the swab to be pushed off the shaft by the use of he coaxially mounted tube. Yet another embodiment is provided with dual specimen collectors to allow simultaneous collection of identical specimens onto separate swabs. Yet another embodiment provides a reagent vial cap retaining stand or projection to provide a specific, reproducible storage location for placement of the vial cap to avoid introduction of the cap into the crime scene by an investigator removing the cap from a reagent vile and placing the cap on a surface that in or adjacent to the specimen to be collected and part of the crime scene.

### Description of the Drawings

Fig. 1 is a top, front and right side perspective view of an embodiment showing the swab removed from the holder and the swab reversed and inserted into the neck of the holder to allow the holder to act as a handle for the swab during specimen collection procedures and showing fixed desiccant retainers holding the desiccant packets at a set distance from the area occupied by the swab when it is inserted into the holder;
Fig. 2 is a bottom, back and left side exploded view of the embodiment of Fig. 1 showing the desiccant chamber cap spaced from the desiccant chamber and two desiccant packets removed from the desiccant chamber and showing within the desiccant chamber the fixed desiccant retainers or guards that hold the desiccant packets at a specific distance from the swab while allowing insertion of desiccant packets into desiccant chamber and showing the swab aligned for insertion into the holder for drying, transport and protection from contamination;
Fig. 3 is bottom, back and left side perspective view of an embodiment showing the swab inserted into the holder where is becomes positioned between the desiccant packets held in the desiccant chamber to permit drying of a specimen collected on the swab during storage and transportation of the swab within swab holder to a laboratory for analysis of the specimen;

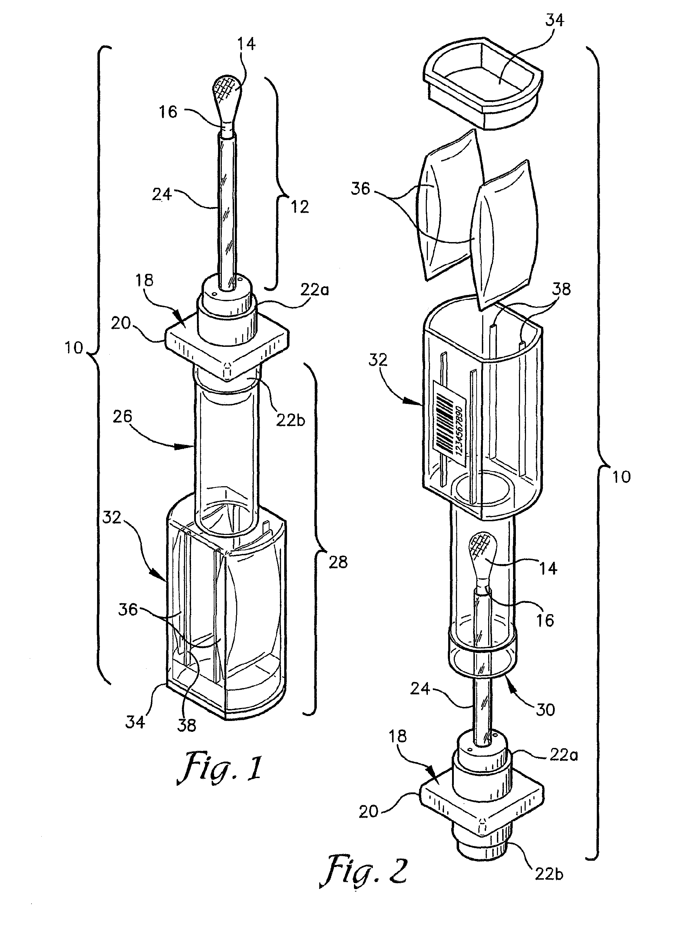

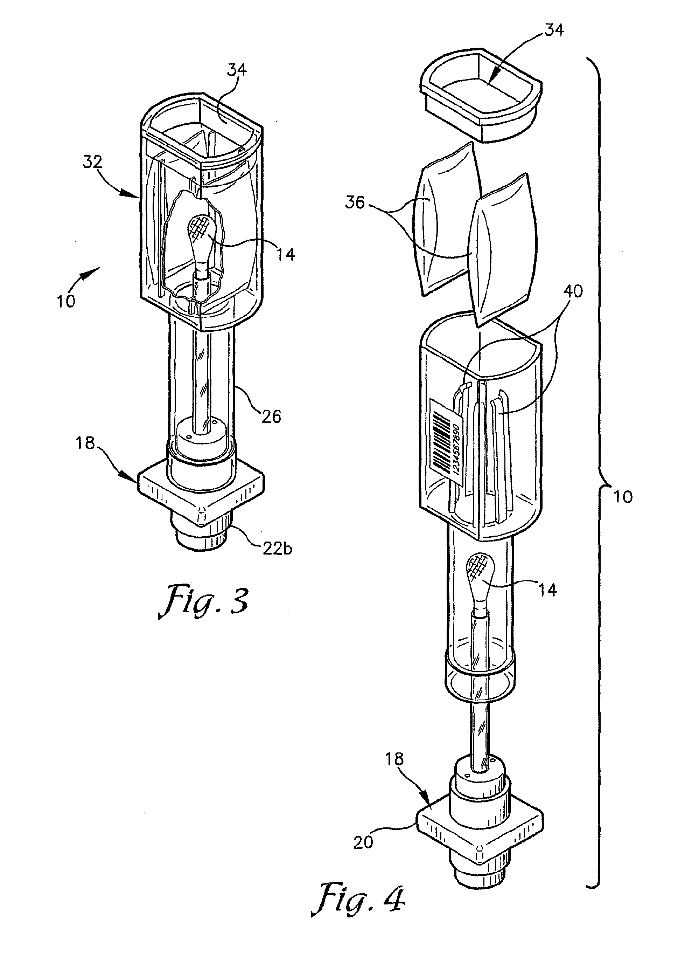

Fig. 4 is a bottom, back and left side exploded view of an embodiment similar to that of Fig. 2, but showing the desiccant being retained by multiple flexible retainers or guards that accommodate desiccant packets of various sizes and allow variation in the distance between the swab and the desiccant packet thereby allowing for variation in the speed of specimen drying and allow for accommodation of specimens of greater volume which may require a larger amount of desiccant in the packets to achieve the desired degree of specimen dryness during transport of the collected specimen in the holder;
Fig. 5 is a bottom plan view of the desiccant chamber of the holder showing the swab positioned within fixed retainers or guards, the retainers or guards being spaced from the swab and any specimen on the swab to keep the specimen out of contact with the desiccant packets during drying and/or transport and/or storage;

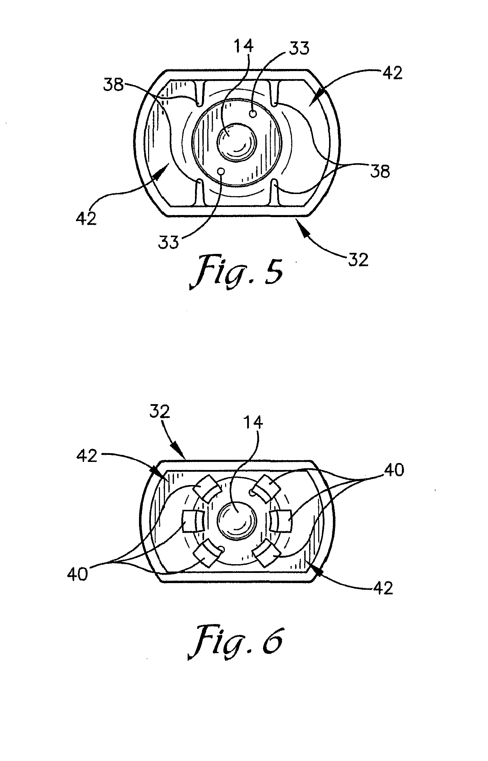

Fig. 6 is a bottom plan view of the desiccant chamber of the holder showing the swab positioned within a set of flexible retainers or guards the retainers being spaced from the swab, but being flexible at the point where the retainers contact the holder to permit the flexible retainers to accommodate variously sized desiccant packets to allow for variations in desired specimen drying time and variations in the specimen liquid content which can affect drying time as well as allowing for variation in the distance of the specimen from the desiccant which can change the drying time during storage and/or transport of the specimen.
Fig. 7 is a top, front and right side perspective view of an embodiment similar to that shown in Figs. 1-6 and having reagent holders mounted thereon;
Fig. 8 is a bottom rear and left side perspective view of a first variation of the device and showing a "T-shaped" securing structure on the bottom of the embodiment for holding a vial to the bottom of the embodiment;
Fig. 9 shows a bottom and front and left side prospective view of a second variation of the device and showing a friction-fit "C-shaped" securing structure for holding a vial to the bottom of the embodiment;
Fig. 10 is a cross-section view taken along line 10-10 of Fig. 9 and showing of the embodiment shown in Fig. 3 with the vial inside the exterior container held by the "C-shaped securing structure and showing the solid construction of central section or central member **20** which may be drilled through if desired to provide gas communication through the closure;
Fig. 11 is a cross-section view taken along line 11-11 of Fig. 7 and showing reagent vials within the reagent holders and also showing the solid construction of central section or central member **20** which may be drilled through to provide gas communication through the closure;
Fig. 12 is a left side, front and bottom perspective view of another embodiment showing a reagent vial held in the bottom of the embodiment;
Fig. 13 is a cross-section view taken along line 13-13 of Fig. 12 and showing the insertion of the reagent vial into a cavity in the bottom of the device and held there by a frictional fit;
Fig. 14 is a front right side and top perspective view of an embodiment of the embodiment having a vial formed in the sides of the device and a cap thereon with the structure of the embodiment walls also forming the walls of the vial;
Fig. 15 is a cross-section view taken along line 15-15 of Fig. 14 and showing the formation of the vials on the front and back sidewalls of the embodiment and showing the solid construction of central section or central member 20 which may be drilled through to provide gas communication through the closure;
Fig. 16 is a front, right side and top perspective view of an alternate embodiment of the embodiment of Fig. 1 and having a vial and cap insert that can be placed into a securing sleeve on the embodiment and having a cap receptacle for holding the vial cap to avoid contamination of a crime scene through the introduction of external materials into the crime scene such as the cap that closes the vial of the present embodiment;
Fig. 17 is a front, right side and top perspective view of the embodiment of Fig. 22 and showing the cap removed from the vial and placed on the cap receptacle to hold the vial cap to avoid contamination of a crime scene through the introduction of external materials into the crime scene such as the cap that closes the vial of the present embodiment;
Fig. 18 is a front, right side and top perspective view of an alternate embodiment and showing the cap receptacle for holding the vial cap included as part of the cap that seals the body of the container;
Fig. 19 is a front, right side and top perspective view of the embodiment of Fig. 18 and showing the cap removed from the vial and placed on the cap receptacle that is positioned on the cap that seals the body of the container;
Fig. 20 is a front and top perspective view of the vial and cap insert that may be used with the embodiment of Figs. 24 and 25 and other embodiments;
Fig. 21 shows an embodiment having dual swabs on dual shafts with each shaft having a break-off tube coaxially mounted on the shaft to allow for simultaneous, dual specimen collection by a user and showing the alignment indicator and closure rotation lock on the closure and on the holder that allows the user to properly align the dual swab collector on the holder to provide proper spacing of the swabs from the desiccant and showing dual vial carriers made integrally with the body of the device and showing a closure rotation indicator and locking structure on the neck of the embodiment;
Fig. 22 shows a cross-section view of the embodiment of Fig. 21 taken along line 22-22 of Fig. 21 and showing the neck of the embodiment of Fig. 22 having the closure rotation indicator and locking structure **74** on the neck of the embodiment engaged with the closure rotation lock **75** on closure **18;**
Fig. 23 is a front and top perspective view of a vial and cap insert that may be used with the embodiment shown in Figs. 27 and 28 and other embodiments;
Fig. 24 shows an exploded perspective view of an embodiment of a closure which can be used with the embodiments described herein and having a reagent vial insertable into the closure for transport of a swab solution therein;
Fig. 25 shows a swab being separated from the shaft by use of a break-off tube coaxially mounted on the swab shaft.

### Detailed Description

As required, detailed embodiments of the present inventions are disclosed herein; however, it is to be understood that the disclosed embodiments are merely exemplary of the invention, which may be embodied in various forms. Therefore, specific structural and functional details disclosed herein are not to be interpreted as limiting, but merely as a basis for the claims and as a representative basis for teaching one skilled in the art to variously employ the present invention in virtually any appropriately detailed structure.

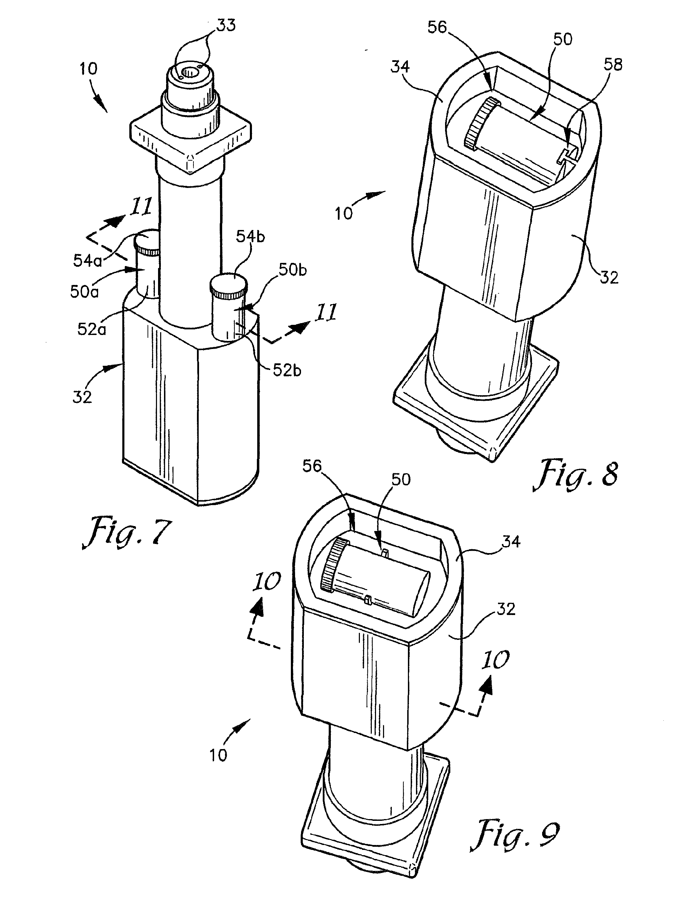

Figures 1 - 24 relate to embodiments of a unitized apparatus for collection and/or drying and/or transport and/or analysis apparatus 10 and a method for modulating drying time of the specimen through the use of user selectable and user sizeable desiccants and user renewable desiccants. Apparatus **10** comprises, generally, a swab mounted on a shaft, the shaft connected to a closure, and a housing or holder having a drying chamber containing a desiccant. The embodiments shown in Figs. 1-6 are generally similar in construction but different in the means by which the desiccant is retained within the holder. The embodiments of Figs. 7-19 include quantified reagent holders.

First referring to Figs. 1-4 the unitized apparatus for collection and/or drying and/or transport and/or analysis apparatus **10** will be described. In Fig. 1 specimen collector **12** comprises a swab **14** mounted on a first end of a shaft **16** with the second end of the shaft connected to a closure **18.** The closure **18** comprises a central member 20 having a stopper **22a, 22b** extending from each of the two opposed sides of the central member. The specimen collector **12** further comprises a break-off tube **24** mounted coaxially on the shaft **16.** The tube **24** is formed of a material that has greater rigidity than the material used to form shaft **16.** A first end, of tube **24** is connected to closure **18** and a second end of tube **24** is configured to terminate at a selected location along shaft **16** at which it is desired to break shaft **16** to achieve separation of swab **14** and the portion of the shaft to which swab **14** is mounted from the remainder of shaft **16.** This location on shaft **16** is referred to as the break-point location and will vary depending on the length of break-tube **24** that is mounted on shaft **16.** Alternatively the break-tube **24** may be connected into closure **18** in a separable manner to allow tube **24** to be pulled from connection with closure **18** and pressed along or slid along shaft **16** until it contacts swab **14** whereupon it can be used to force swab **14** off of shaft **16** and into a container or other receptacle.

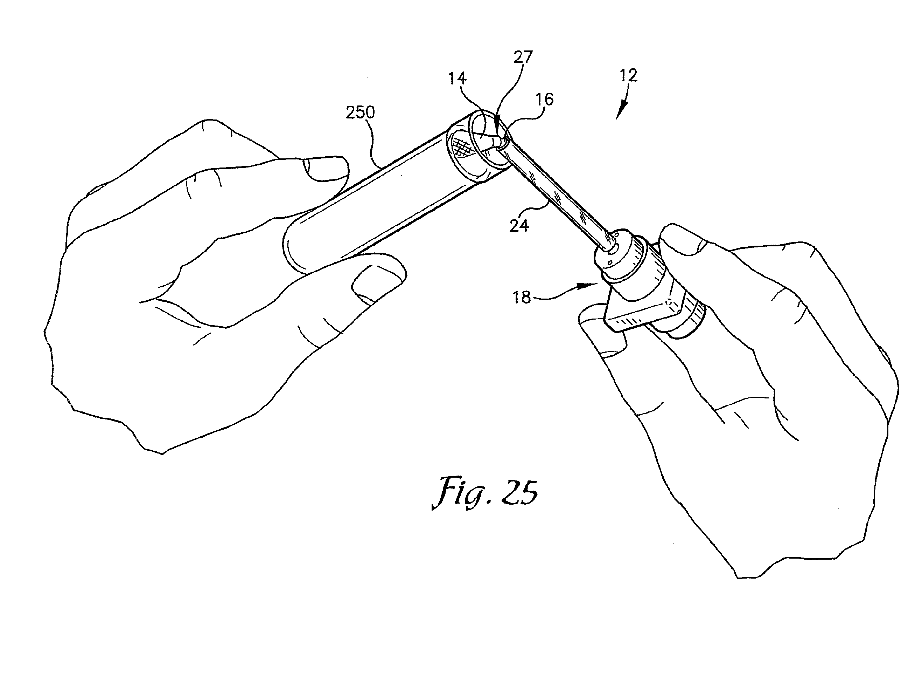

For clarity this type of separation of swab **14** from specimen collector **12** is shown in Fig. 25. In Fig 25, it may be seen that swab **14** is pressed against a solid surface such as the side of container **250** and a bending motion is applied by the user to press swab **14** back toward tube **24** and closure **18.** Upon sufficient pressure being applied, the shaft **16** will break at or near the terminus of tube **24.** Then swab **14** and the portion of shaft **16** to which swab **14** is connected will separate from the portion of shaft **16** that is connected to closure **18.** This allows the swab and the specimen that is collected onto the swab to be separated from the remainder of device **10** and separately placed into a reaction tube for analysis and/or an alternate container for shipment.

Again referring to Fig. 1 it will be appreciated that closure **18** comprising central member **20** and having a stopper **22a, 22b** extending from each of the two opposed sides of the central member is shown with stopper **22a** having shaft **16** and tube **24** connected thereto and with stopper **22b** inserted into neck **26** of housing **28** of apparatus **10.** Fig. 1 presents the embodiment in its open position. In the open position, specimen collector **12** has been removed from housing **28** and the closure **18** has been reversed and inserted into opening **30** (Fig. 2) of the neck **26** of housing **28** from which closure **18** and swab **14** on shaft **16** and break-off tube **24** were just removed. This reversal and insertion allows housing **28** to act as a handle for manipulating the swab **14** of specimen collector **12** during the collection of a specimen onto swab **14.** The relatively large, flat surface of desiccant chamber **32** fits securely into the palm of the hand and provides a flat surface that will prevent rolling of the apparatus **10** if it is placed on a surface. When positioned on a surface the edge of closure **18** extends laterally beyond swab **14** and keeps swab **14** separated from any contact with adjacent contaminating surfaces. The closed position for apparatus **10** is shown in Fig. 3 wherein specimen collector **12** has been inserted into housing **28** and stopper **22a** of closure **18** has been inserted into opening **30** (Fig. 2) of the neck **26** of housing **28** so that stopper **22a** of closure **18** having swab **14** on shaft **16** and break-off tube **24** connected thereto all are inserted into housing **28.**

Referring now to Figs. 2 and 4 the housing **28** being further comprised of desiccant chamber **32** connected to neck **26** of housing **28,** will be described. Desiccant chamber **32** is provided with resealable cover **34** that forms the bottom of **housing 28.** Cover **34** may be generally flat to allow apparatus **10** to stand on a surface. Cover **34** may be removably connected to desiccant chamber **32** or it may be permanently sealed to close chamber **32.** It will be appreciated that the permanent sealing of chamber **32** by cover **34** may be accomplished at the time of manufacture or upon the insertion of a specimen on swab **14** into housing **28** or cover **34** may be used to permanently seal housing **28** at any time thereafter. Desiccant packets **36** of Figs. 2 and 4 have been removed from desiccant chamber **32** to better show the fixed retainers **38** (Fig. 2) and flexible retainers **40** (Fig. 4) that hold desiccant packets **36** in position within desiccant chamber **32.** It will be appreciated from the Figs. 2 and 4 that fixed retainers extend from a sidewall of desiccant chamber **32** and flexible retainers are a plurality of flexible finger-like structures that extend downwardly from the top of desiccant chamber **32** and can accommodate desiccant packets of various sizes and shapes by flexing toward and away from the swab isolation area **41.** When swab **14** is positioned within housing **28,** as shown in Fig. 3, it may be seen that swab **14** situated between the retainers **38** (Fig. 2) or within retainers **40** (Fig. 4) in a swab isolation area **41** with the retainers **38, 40** holding desiccant packets **20** away from swab **14.** It will be appreciated that swab **14** is positioned between, but not contacted by, desiccant packets **36** to avoid contamination of swab **14.**

In Fig. 4 an embodiment similar to that of Fig. 2 is shown in an exploded view. In Fig. 4 desiccant chamber cover **34** is separated from the desiccant chamber **32** and the two desiccant packets **36** have been removed from the desiccant chamber **32.** Visible within the desiccant chamber **32** are the flexible retainers **40** that allow variable spacing of the desiccant packets **36** from the swab **14.** It will be appreciated that the flexible nature of flexible retainers **40** allows insertion of variously sized desiccant packets **36** into desiccant chamber **32.** This is accomplished by the flexible retainers **40** being able to bend inwardly toward swab **14** to expand the distance between flexible retainers **40** and the walls comprising desiccant housing **32.** Due to this repositionable nature of flexible retainers **40,** user selectable quantities of desiccant and variable volumes of desiccant and variable sizes of desiccant packets can be introduced by the user into desiccant chamber **32** to change the drying time of a specimen captured on swab **14.** Desiccant packets **36** are positioned to be in close proximity to swab **14** to absorb moisture from the specimen that is collected on swab **14.** As the proximity of desiccant to moisture has a direct correlation to the rapidity of drying, it will be appreciated that the close, but spaced, proximity of the desiccant to swab **14** is particularly efficacious in speeding the drying of moisture that may be on swab **14.** Such variation is made possible by flexible retainers **40.** It also will be appreciated that resealable cover **34** permits the replacement of desiccant packets **36** at anytime during the use of device **10** and without the need to disturb swab **14** and/or any specimen thereon.

In Fig. 3 a perspective view is shown of the device **10** of Figs. 2 and 4 with swab **14** inserted into housing **28.** In this position swab **14** is positioned between desiccant packets **34** for drying and is protected within housing **28** for transport and/or storage. It may be observed that swab **14** is positioned between guards **38.** In Fig. 3 a portion of the desiccant packets **36** have been removed and a portion of the wall of desiccant chamber **32** has been removed for clarity.

It will be understood that in Fig. 3, closure **18** has been reinserted into neck **26** to dispose swab **14** and shaft **16** and break-off tube **24** within housing **28.** This positioning places swab **14** disposed between retainers **38, 40** and within desiccant chamber **32.** It will be appreciated that flexible retainers **40** extend beyond the bottom of swab **14** to prevent objects inserted into desiccant chamber **32** from making inadvertent contact with swab **14.** Those skilled in the art will appreciate that with desiccant chamber cover **34** removed, as shown in Fig. 4, that desiccant chamber **32** is open and accessible. It is in this configuration that desiccant packets **36** can be inserted, removed, renewed or increased or decreased in size by the user as may be indicated by the needs of the particular specimen on swab **14** or the need to speed up or slow down drying of the specimen on swab **14.** It also may be observed in Figs. 5 and 7 that closure **18** may be provided with air holes **33** that extend through closure **18.** Air holes **33** can aid in the drying of the specimen and air holes **33** can be excluded from the embodiment completely if desired.

In Fig. 5 the fixed or rigid retainers **38** and the swab **14** are shown from a bottom view into desiccant chamber **32.** In this view it may be seen that swab **14** is positioned between retainers **38** and spaced therefrom so as not to contact retainers **38** or the walls of desiccant chamber **32.** Desiccant holding areas **42** extending between retainers **38** and the walls of desiccant chamber **32** are best observed in Figs. 5 and 6. It will be appreciated that variously sized desiccant packets **36** can be inserted into desiccant holding areas **42** during drying and/or transport and/or storage. Once the desiccant packets **36** have become exhausted by absorption of moisture they may be replaced. This is accomplished by removing cover **34** withdrawing exhausted desiccant packets **36** and inserting new desiccant packets **36.** Once replacement has been accomplished, the desiccant chamber resealable cover **34** may be replaced to again close desiccant chamber **32** to the outside.

In Fig. 6 the flexible guards **40** and the swab **14** are shown from a bottom view into desiccant chamber **32.** In this view it may be seen that swab **14** is positioned within flexible guards **40** and spaced therefrom so as not to contact flexible guards **40** or the walls of desiccant chamber **32.** It will be appreciated that the ends of flexible guard **40** bend inwardly to operate to deflect material, such as desiccant packets **36** when they enter desiccant chamber **32,** from contacting swab **14** and any specimen thereon. Desiccant holding areas **42** extending between flexible guards **40** and the walls of desiccant chamber **32.** It will be appreciated that as flexible guards **40** may be pushed away from desiccant chamber **32** walls that variously sized desiccant packets **36** can be inserted into desiccant holding areas **42 during** drying and/or transport and/or storage. Once the desiccant packets **36** have been inserted, the desiccant chamber resealable cover **34** may be replaced to again close desiccant chamber **32** to the outside. It will be appreciated that the flexible guards **40** in particular allow the user to select and insert variously sized desiccant packets to modulate the drying time of the collected specimen or sample depending upon user desires for the particular specimen or sample. In addition the flexible guards **40** permit larger desiccant packet volumes to approach more closely to the swab 14 as it resides in the swab isolation area 41 since the flexible guards 40 can move inwardly toward the swab thereby placing the desiccant closer to the specimen. This configuration will modulate the drying of the specimen as the closer proximity of the desiccant to the moisture of the specimen on the swab will decrease the drying time of the specimen and enhance the stability of the collected specimen by drying the specimen faster.

Referring now to Fig. 7 an embodiment of a type shown in Figs. 1-6 is shown further comprising the addition of reagent holders mounted on the top of desiccant chamber **32.** Reagent holders **50a, 50b** extend from desiccant chamber **32** and are molded in unitary fashion with desiccant chamber **32.** The reagent holders **50a, 50b** are comprised of a body 52a, **52b** and a cap **54a, 54b.** Caps **54a, 54b** may be of the screw type or the friction fit type of cap.

Referring now to Fig. 8 and Fig. 9, embodiments are shown having the reagent holders **50** mounted on desiccant chamber removable cover **34.** In the embodiment of Fig. 8, reagent holder **50** is held within an indention formed in cover **34.** The indention being sufficient to allow the entirety of reagent holder 50 to sit within the indention while yet allowing apparatus **10** to stand on a flat surface with resalable cover 34. Such contact with the surface is shown in Fig. 7. In Fig. 8, reagent holder **50** is retained within indention **56** by a tongue and groove shaped arrangement with the groove being within the bottom of the reagent holder **50** and the tongue extending from removable cover **34** and being configured to be mateable with the groove in the bottom of the reagent holder **50.** In Fig. 9, the reagent holder **50** is retained within indention **56** by C-shaped which provides a frictional fit capture of the reagent holder **50** within the C-shaped retaining clip.

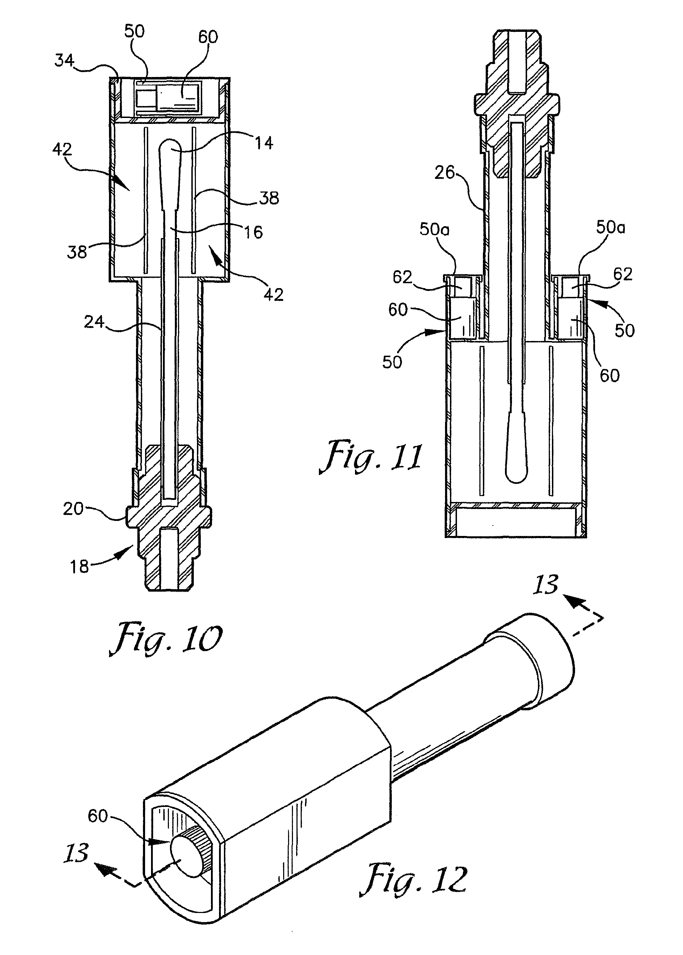

Referring now to Fig. 10, a cross-section view taken along line 10-10 of Fig. 9 is shown. In Fig. 10, it can be seen that a device of similar construction to the device shown in Figs. 1 and 2 is shown having desiccant holding areas **42** and retainers **38** and a swab **14** on shaft **16** having tube **24** coaxially mounted thereon. Also shown in Fig. 10 is reagent vial **60** which is in reagent holder **50.** It will be appreciated by those skilled in the art that using a separate reagent vial **60** held within a reagent holder **50** that different reagent compositions and of different volumes may be rapidly and easily substituted into reagent holder **50** by simple substitution of a different reagent vial **60.**

Referring now to Fig. 11, a cross-section view taken along line 11-11 of Fig. 7 is shown. In Fig. 11, reagent holders **50** are shown to either side of neck **26** with each vial **60** having a cap **62** thereon and reagent holder **50** having its own cap **50a** serving to retain vial **60** within reagent holder **50.**

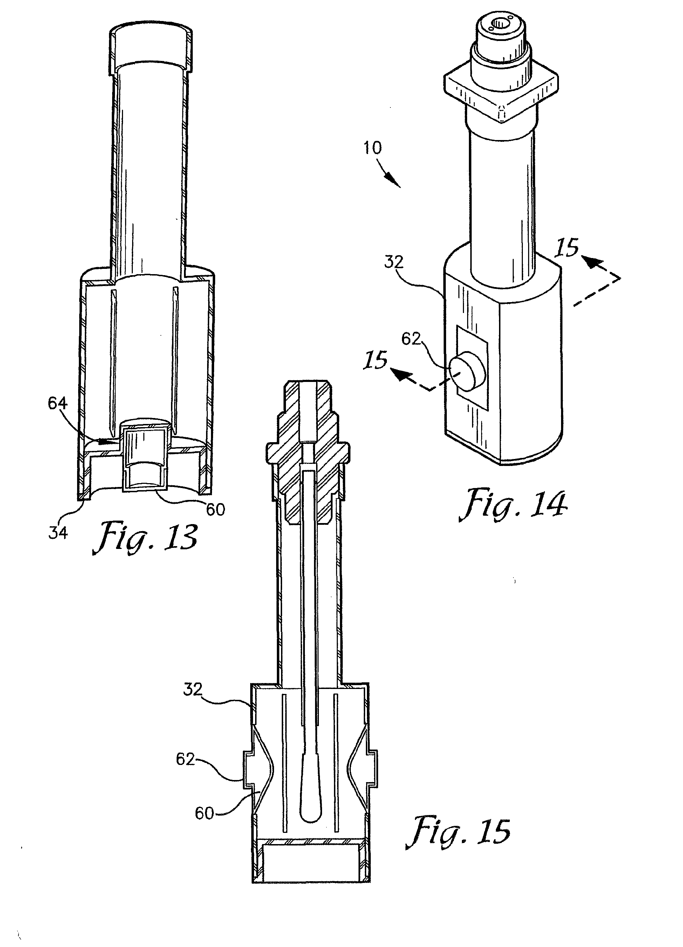

Referring now to Fig. 12, an embodiment is shown having reagent vial 60 inserted into a depression formed in the surface of desiccant chamber receivable cover 34. In Fig. 13, a cross-section view taken along line 13-13 of Fig. 12 is shown. In Fig. 13, the cross-section view of the embodiment of Fig. 12 shows that cover **34** is provided with an indention **64** which is configured to capture vial **60** therein by a frictional fit between the bottom of vial **60** and the walls of indention **64.**

In Figs. 14 and 15, yet another embodiment of the reagent holder on the apparatus is shown. In Fig. 14, it can be seen that the reagent vial **60** is formed integrally with the sidewall of desiccant chamber **32.** This may be more clearly seen in Fig. 15, which is a cross-section view taken along line 15-15 of Fig. 14. In Fig. 15, reagent vial **60** is shown as comprising an indention in the sidewall of desiccant chamber **32** and having cap **62** thereon to seal reagent vial **60.**

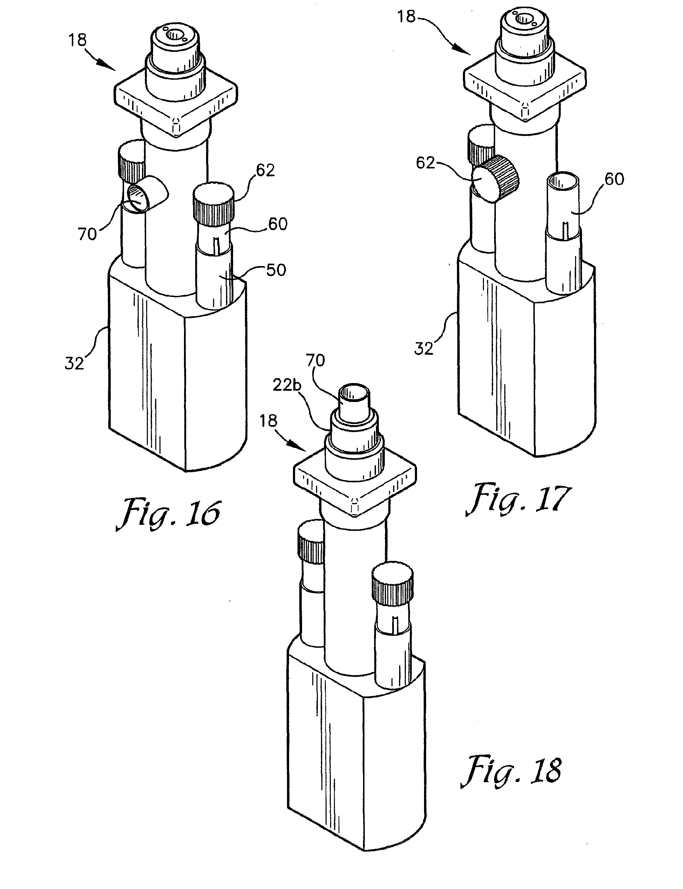

Referring now to embodiments shown in Figs. 16-20, embodiments having reagent holders and reagent vials are shown but also having the added advantage of having a cap stand included in the embodiment to retain a reagent holder cap or a reagent vial cap and to provide secure, reproducible placement in the keeping of the reagent or vial cap thereby to avoid loss of the vial cap and to avoid contamination of a crime scene in particular. The cap receptacle allows the evidence collection technician to avoid contamination of a crime scene by the inadvertent introduction of external materials into the crime scene. Specifically, the receptacle allows the cap that closes the vial to be placed in a specific, anticipated, repeatable location that is a part of the equipment brought to the scene by the evidence collection technician. In this manner the evidence collection technician will always know where to put the cap and where to locate it at the conclusion of the specimen collection. This provides a consistent and repeatable activity that can become a part of the evidence collection technicians method of practice and thereby reduce the introduction of external materials and potential extraneous DNA that might contaminate the crime scene.

Referring now to Fig. 16 and 17, an embodiment is shown having a cap stand **70** extending from neck **26** of holder **28.** In Fig. 17, it can be seen that a cap **62** has been removed from reagent vial **60** and has been placed onto cap holder **70** where cap **62** is retained during the course of a collection procedure performed with the embodiment shown in Fig. 17. It also will be appreciated that having the reagent holder **50** and reagent vial **60** positioned in upright fashion on the top of desiccant chamber **32** allows the investigator, particularly a crime scene investigator, to have the reagent contained in reagent vial **60** available for use in wetting the swab **14** which is attached to closure **18** without a need to attempt to manipulate additional devices and structures to wet the swab **14** or to find a suitable location to place holder **28** within the crime scene to free a hand to hold the reagent vial **60** while wetting swab **14** of a specimen collector **12** with a suitable reagent such as that which is contained in reagent vial **60** for a specimen collection.

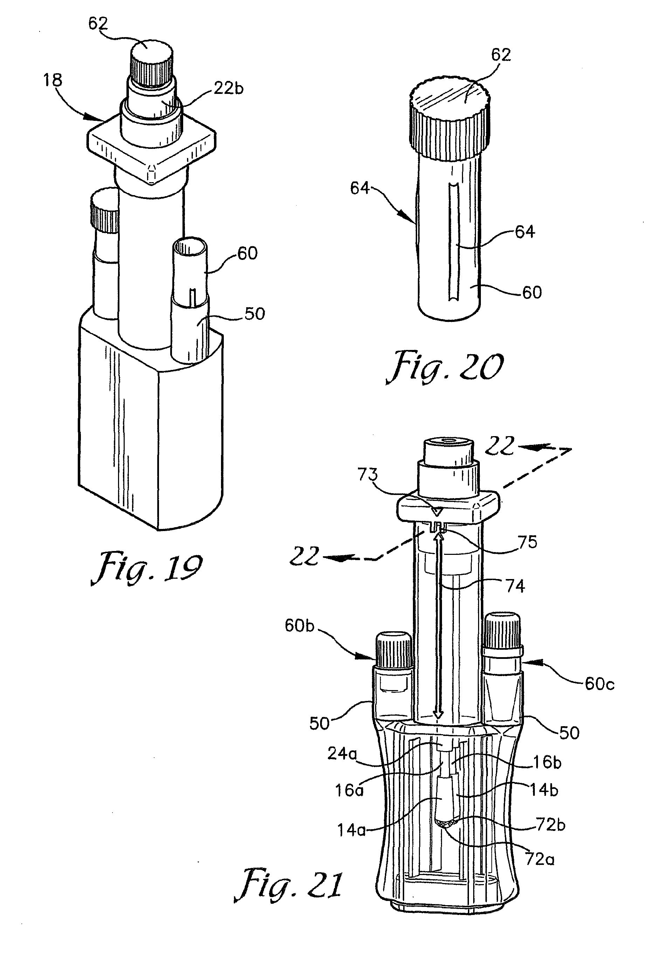

Referring now to Fig. 18, an alternate embodiment is shown and which is similar to the embodiments of Figs. 16 and 17 but in which the cap stand **70** is formed in the top of stopper **22b** of closure **18.** It will be appreciated that the embodiment of Fig. 18 operates in similar manner to the embodiment described in Fig. 16 and 17. Such similar operation is shown in Fig. 19 wherein a cap **62** has been removed from a vial **60** and the cap **62** has been placed upon cap stand **70** which extends from stopper **22b** closure **18.** In Fig. 20, reagent vial **60** is shown of the type used in many of the embodiments described herein. Vial **60** is provided with longitudinal projections **64** which are compressible and which enhance the friction fit of reagent vial **60** within reagent holder **50** and which allow the passage of air in and about the sidewall of reagent vial **60** and the sidewall of reagent holder **50** when the two are insertably joined together as shown in Fig. 19. The importance of this feature will be appreciated by those skilled in the art who have contended with a moisture seal between two closely fitted surfaces and the barrier to separation of the two structures caused by the moisture seal preventing the intrusion of air and causing a need to overcome a vacuum which is created between the two surfaces when the withdrawal of the objects from insertion, one within the other, is attempted. Projection **64** assists in such separation while also providing secure frictional fit between vial **60** and reagent holder **50.**

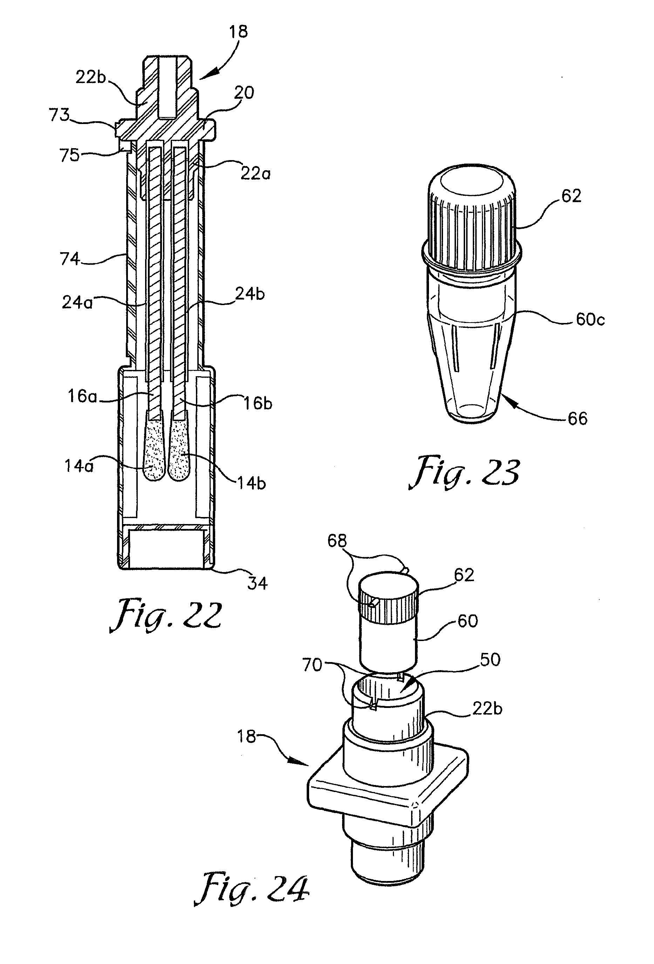

In Figs. 21 and 22 an embodiment is shown having dual swabs **14a,b** mounted on dual shafts **16a,b** and having dual break-off tubes **24a,b** coaxially mounted on each of the shafts. Both of these dual swab, shaft and break-off tube combinations are connected to the same stopper **22a** extending from central member 20 of closure **18.** The embodiment of Figs. 21 and 22 allows the user to collect simultaneously, identical, dual specimens or samples **72a,b** in one motion or in a single contact with a specimen or evidence location. Then the user can simultaneously deliver the dual and identical specimens **72a,b** to a single housing **28** to thereby assure that the specimen or evidence samples receive simultaneous and identical protection and drying conditions and transport conditions are provided to the identical, dual specimens. The embodiment of Figs 21 and 22 permits a user to remove one of the two identical and simultaneously collected specimens **72a,b** while allowing the other specimen or sample **72a,b** to remain untouched or unused and to be archived without removal of the specimen from the original housing into which it was inserted after collection. This simultaneous, dual collection and protection of a specimen or evidence sample is of great importance for evidence collection as it allows collection of two identical specimens **72a,b** under exactly the same conditions, from exactly the same location of the evidence, and permits the separate removal and testing of one of the dual identical specimens without any change or disturbance to the other specimen and while leaving one of the dual identical specimen fully intact and untouched for archiving and further or future testing. This can be highly important in providing a second identical specimen for test verification where an analysis method that is destructive of the specimen must be employed.

Fig. 21 an embodiment is shown having dual swabs **14a,b** connected to dual shafts **16a,b** and with each shaft having a break-off tube **24a,b** coaxially mounted on the shaft. As previously described for Fig. 25, the break-off tubes **24a,b** allow for the swab **14** to be separated from the shaft **16.** It also will be appreciated that the embodiment of Figs. 21, 22 is provided with desiccant packets **36** in desiccant chamber **32** to permit simultaneous, and identical drying conditions for the dual specimens. As previously described, when swabs **14a,b** are positioned within housing **28,** the swabs **14a,b** are to be situated between the retainers **38** (Fig. 2) or within retainers **40** (Fig. 4) with the retainers **38, 40** holding desiccant packets **20** away from swab **14.** It will be appreciated that it is important that swabs **14a,b** be positioned between, but not contacted by, desiccant packets **36** to avoid contamination of swabs **14a,b.** To assure the proper location of swabs **14a,b** the embodiment of Figs. 21, 22 is provided with alignment indicators on closure **18** and holder **28.** In Figs. 21 and 22 closure **18** is provided with indicator 73 on central member **20** and holder **28** is provided with indicator **74.** In operation, a user upon inserting specimen collector **12** into holder **28** will observe the alignment of indicators **73** and **74** and then rotate closure **18** within holder **28** until the indicators **73, 74** are aligned one above the other as shown in Fig. 21. This alignment assures that the swabs **14a,b** are positioned between retainers **38** or **40** in a position that provides uniform separation between each of swabs **14a,b** and desiccant packets **20.** In this manner the identical drying of swabs **14a,b** is assured.

The embodiment of Figs. 21 and 22 also includes a closure rotation lock **75** on the closure **18.** During insertion of specimen collector **12** into holder **28** and after alignment of indicators **73, 74** the closure can be pressed downwardly into holder **28** to insert holder indicator **74** into closure rotation lock **75** to thereby prevent inadvertent rotation of specimen collector **12** within holder **28.** In this manner the proper alignment of the dual swab collector on the holder to provide proper spacing of the swabs from the desiccant is assured during future use and transportation.

It will be appreciated that the embodiment of Fig. 21 and 22 can be used to capture evidence at a crime scene that may be used as a control during analysis while providing exactitude in the identical handling of the control swab since both the control swab and the specimen swab are handled simultaneously during the collection and drying and transport phases of evidence collection and the evidence security will be identical for both specimens. In the case that one of the dual swabs may be a control the evidence collector would use a first of the two dual swabs to take a specimen of the area surrounding the evidence specimen of interest. Then the second swab would be used to obtain a sample of the evidence specimen as it existed in the crime scene. Then both swabs would be treated identically and simultaneously during the remainder of the collection and insertion into the housing and marking and evidence security and shipping procedures. If a specimen containing DNA was collected on the evidence swab, the control swab could be examined to determine if background DNA was present in the vicinity of the DNA evidence and if background DNA was present on the control swab the background DNA then could be removed from the analysis of the DNA found on the evidence swab.

A further use of the dual swab embodiment may be to provide simultaneous, wet/dry specimen collection from a single evidence specimen after which both swabs may be treated identically and simultaneously during the remainder of the collection and insertion into the housing and marking and evidence security and shipping procedures. In this method of collection one of the dual swabs is wetted with a reagent contained in one of the reagent vials **60** which contains a wetting reagent therein. The wetted swab is then applied to the evidence of interest and used to both collect a wetted sample from the evidence. This procedure will result in a wetted evidence of interest after which the other swab of the dual swabs, the dry swab, may be applied to the now wetted evidence of interest to further collect a sample of the evidence of interest.

Also shown in Fig. 21, the provision for both a reagent holder **50** and a separate reagent vial **60** will be appreciated for allowing the use of variously sized reagent vials **60** which can contain precisely measured but different volumes of reagent to be applied to either swab **14** or to a specimen to be collected. As shown in Fig. 21, vial **60b** is substantially smaller than is vial **60c.** In providing individual vials for the provision of reagents to be applied to swab **14,** the benefit is provided that exact quantization of the dilution of a specimen that is collected can be determined. In the prior art typical swab wetting procedure, an absorbent swab is held beneath a container nozzle and the technician attempts to apply individual drops of a reagent to the swab. The usual result is that the first drop or drops or substantial portions thereof bead up and fall off the swab due to the swab surface not being immediately absorbent. In the present embodiments, by providing an actual vial holding a reagent, the swab can be dipped into the vial where the pre-measured optimum quantity of a user selected reagent is held in contact with the swab **14** and complete absorption of the reagent onto the swab is accomplished. This absorption is further assisted by the pressure that can be brought to bear on the swab by the sidewalls of the vial **60** pressing against the swab **14** to assist in overcoming the surface tension present on the swab **14** thereby assisting in overall absorption of the reagent contained in vial **60.** In Fig. 23, a vial of the type shown inserted in the reagent holder **50** of Fig. 21 is shown in greater detain and having inverted conical sidewalls **66** which further assists in the complete absorption of a small volume of reagent liquid on to swab **14.** It will be appreciated that depending on what specimen is to be collected or what specimen is of interest to the investigator that the quantity and type of reagent in the vial may be user selected. For example if it is of particular interest the semen be immediately identified if it is present in the crime scene then the user or evidence technician can insert vials into the reagent holders that contain a semen reactive reagent to identify the presence of semen upon the swab contacting semen in the crime scene evidence. Or, if blood is of particular interest the evidence collection technician can insert vials into the reagent holders that contain a blood reactive reagent to identify the presence of blood upon the swab contacting the unknown crime scene specimen.

The quantified reagent vials **60** which are interchangeable within the reagent holders **50** are configured to provide a reproducible, quantitative wetting of the swab with a known amount of solution and which results in the wetting of the swab by a known volume this provides a quantified absorption of reagent onto the swab which is not possible with previous devices. As described above, the past procedures of attempting to add reagent in a drop-wise manner onto the swab could not produce a swab having a known quantity of reagent on the swab due to loss of drops or loss of portions of drops from the swab surface prior to absorption of the drop by the swab.
Fig. 24 shows a closure **18** having a reagent holder **50** formed into a stopper **22b** for insertion of a vial **60** therein and with cap **62** of vial **60** being provided with flanges **68** which are captured within detents **70** of stopper **22b** which assists in drawing vial **60** from stopper **22b** as cap **62** will, when inserted into stopper **22b,** be flush with the top of stopper **22b.**

In Fig 25 the method by which swab **14** is separated from shaft **16** by applying the terminal end of break-off tube **24** to a break-point **27** located on shaft **16.** In Fig. 25 swab 14 is pressed against the side of container **250** and a bending motion is applied by the user to press swab **14** back toward tube **24** and closure **18.** When sufficient pressure is applied shaft **16** will break at or near a break-point **27** which is adjacent the terminus of tube **24** as it is the terminus of tube **24** which establishes to point of application of bending force to shaft **14.** When sufficient force is applied, shaft **16** will break and swab **14,** and the portion of shaft **16** to which swab **14** is connected, will separate from the portion of shaft **16** that is connected to closure **18.** This allows the swab and the specimen that is collected onto the swab to be separated from the remainder of device **10** for analysis and shipment. Alternatively, the break-off tube may be used as a swab pushed-off device. In this instance the break-off tube may be pushed by the user along the shaft to slide the break-off tube into contact with the swab. The break-off tube in this embodiment of configured to be a close, but slideable coaxial fit on the shaft and sufficiently smaller in diameter than the swab that the break-off tube will not slide over the exterior of the swab. In this embodiment the break-off tube will contact the swab and be used by the user to press the swab off the end of the shaft and into a reaction container or other tube or holder or shipping container.

The present invention relates to the following items:
Item 1. A specimen collection and specimen drying and specimen transport apparatus having a housing portion configured to act both as a handle for the specimen collection portion during specimen collection and to act as a transport container during specimen shipping, the housing being configured to provide a desiccant chamber wherein the specimen collected on the specimen collection portion can be dried during storage and shipment of the collected specimen the desiccant chamber allowing the desiccant to be removed and new desiccant inserted, the apparatus comprising:
   a specimen collector comprising:
      a specimen collection swab, said swab connected to a shaft having a first shaft end with said specimen collection swab thereon and a second shaft end connected to a closure, said closure having first and second stopper structures extending from opposed sides of a central member, said first stopper structure connected to the shaft,
      a break-off tube mounted coaxially on the shaft, the break-off tube having a first end connected to said first stopper structure and a second end terminating at a shaft break-point on said shaft, said shaft-break-point being positioned along said shaft at a location sufficiently spaced from said swab to permit the entire swab to be separated from the shaft when the shaft break-point is pressed against the break-off tube,
   a housing comprising
      a desiccant chamber,
      a neck extending from the desiccant chamber the neck having an opening providing communication through the neck and into the desiccant chamber, a swab isolation area in the desiccant chamber the swab isolation area aligned with the neck to permit insertion of the shaft mounted swab through the neck and into the swab isolation area of the desiccant chamber the desiccant chamber having a desiccant holding area on at least one side of the swab isolation area the desiccant holding area configured to retain a desiccant packet therein to absorb moisture from a specimen collected on the swab, and
      an openable and closeable bottom on the desiccant chamber configured to permit removal of the desiccant packet from the desiccant holding area and insertion of the desiccant packet into the desiccant holding area while the swab is in the swab isolation area of the desiccant chamber.
Item 2. The apparatus as claimed in item 1 further comprising retaining structures in the desiccant chamber the retaining structures configured to hold the desiccant packet in the desiccant holding area and away from contact with the swab.
Item 3. The apparatus as claimed in item 2 wherein the retaining structures are rigid flanges extending from opposed sidewalls of the desiccant chamber.
Item 4. The apparatus as claimed in item 1 wherein the desiccant packet contains a quantity of desiccant that is sized to correspond to a user selected swab drying time interval.
Item 5. The apparatus as claimed in item 2 wherein the retaining structures are flexible retainers extending downwardly from a top wall of the desiccant chamber.
Item 6. The apparatus as claimed in item 2 wherein the retaining structures are flexible projections extending upwardly from the openable and closable bottom.
Item 7. The apparatus as claimed in item 1 further comprising a unique indicia on the housing for identification of the apparatus.
Item 8. The apparatus as claimed in item 1 further comprising a reagent vial holder connected to the exterior of the desiccant chamber, the holder having a cylindrical shape and extending upwardly from a shoulder area of the chamber, the chamber shoulder area extending outwardly from the connection of said neck to said desiccant chamber.
Item 9. The apparatus as claimed in item 8 further comprising a vial for insertion into the reagent vial holder the vial configured to hold a known volume of a reagent for application to the swab.
Item 10. The apparatus as claimed in item 9 comprising a vial cap holder extending from the outside of the desiccant chamber.
Item 11. The apparatus as claimed in item 8 wherein the reagent vial holder is a "T" shape projection extending from the desiccant chamber resealable cover and the vial comprises a "T" shaped void registrable with the "T" shape projection.
Item 12. The apparatus as claimed in item 11 wherein the vial holder is a "C-shaped" clamp having friction fit with said vial.
Item 13. The apparatus as claimed in item 11 wherein the vial holder is a indention in the desiccant chamber cover.
Item 14. The apparatus as claimed in item 8 wherein said vial holder is formed integrally with a sidewall of said drying section of said holder.
Item 15. The apparatus as claimed in item 8 further comprising a projection extending from the neck the projection being configured to receive a vial lid thereon to retain the lid when it is removed from the reagent vial.
Item 16. The apparatus as claimed in item 8 further comprising a projection extending from the closure the projection being configured to receive a vial lid thereon to retain the lid when it is removed from said reagent vial.
Item 17. The apparatus as claimed in item 1 having void in the second stopper structure of the closure, the void configured to receive a reagent vial therein.
Item 18. The apparatus as claimed in item 16 further comprising projections extending from the lid of said reagent vial the projections extending outwardly beyond the sidewall of said closure to permit the projections to be grasped by a user for extraction of the vial from the void.
Item 19. The apparatus as claimed in item 9 further comprising a selection of vials for insertion into the vial holder said vial selection comprising vials having different reagents compositions and different reagent volumes for substitution into said vial holder to configure the apparatus for different specimen collection applications.
Item 20. A method of specimen collection comprising the steps of:
   providing a specimen collector device comprising at least two specimen collectors extending from a first side of a container closure, each of the specimen collectors comprising
   a specimen collection swab, the swab connected to a shaft having a first shaft end with the specimen collection swab thereon and a second shaft end connected to the container closure, the closure having first and second opposed sides of a central member, the specimen collector swabs being configured to simultaneously contact a specimen to collect a specimen sample on the swab,
   a generally rigid tube mounted coaxially on the shaft, the tube having a first end connected to said first stopper structure and a second end terminating at a shaft break-point on said shaft said shaft-break point being positioned along said shaft at a location sufficiently spaced from said swab to permit the entire swab to be separated from the shaft when the shaft is broken at said shaft break-point,
   providing a housing for holding and drying and transporting the specimen collection swabs the housing comprising
   a desiccant chamber,
   a neck extending from the desiccant chamber the neck having an opening providing communication through the neck and into the desiccant chamber, a swab isolation area in the desiccant chamber the swab isolation area aligned with the neck to permit insertion of the shaft mounted swab through the neck and into the swab isolation area of the desiccant chamber the desiccant chamber having a desiccant holding area on at least one side of the swab isolation area the desiccant holding area configured to retain a desiccant packet therein to absorb moisture from a specimen collected on the swab, and
   an openable and closeable bottom on the desiccant chamber configured to permit removal of the desiccant packet from the desiccant holding area and insertion of the desiccant packet into the desiccant holding area while the swab is in the swab isolation area of the desiccant chamber,
   applying simultaneously both swabs to a specimen,
   collecting simultaneously a sample of the specimen onto both swabs.
   inserting simultaneously both swabs into the neck of the housing to place the swabs in the swab isolation area of the desiccant chamber, and
   drying both swabs simultaneously within the desiccant chamber.
Item 21. The method of item 20 wherein a first swab is applied to the specimen and a second swab is simultaneously applied to an area adjacent the specimen but which does not include the specimen to simultaneously obtain a sample of the specimen and a sample of the area adjacent the specimen.
Item 22. The method of item 21 further comprising the steps of:
   analyzing the sample of the specimen for data regarding the specimen to provide specimen data,
   analyzing the sample of the area immediately adjacent the specimen for data regarding the area, to provide area data, and
   subtracting the area data from the specimen data to obtain specimen specific data.
Item 23. The method of item 20 wherein the housing further comprises
   providing a flexible desiccant retainer to separate the desiccant holding area from the swab isolation area,
   inserting a desiccant packet into the desiccant holding area, and
   permitting the flexible desiccant retainer to move toward or away from the swab isolation area to allow accommodation of the volume of the inserted flexible desiccant packet.
Item 24. A method of pre-wetting a swab with a pre-measured user selected specimen collection reagent comprising the steps of
   providing a specimen collector having specimen collection swab and a reagent holder thereon,
   inserting a reagent vial into said reagent holders, said reagent vial having a premeasured volume of a user selected specimen collection reagent therein,
   inserting the swab into the reagent vial,
   absorbing completely the pre-measured volume of the reagent from the vial onto the swab to provide a swab having a reproducible amount of a reagent thereon, and
   applying the swab having the completely absorbed pre-measured reagent thereon to a specimen for collection of the specimen on the swab.
Item 25. A method of avoiding contamination of an evidence collection scene the method comprising the steps of:
   providing a specimen collector having a reagent holder thereon,
   inserting a reagent vial into said reagent holder, said reagent vial having a cap thereon to close the vial,
   providing a projection on the specimen collector the projection configured to hold the cap by either friction fit or by threaded connection,
   removing the cap from the vial during specimen collection at a evidence collection scene, and
   connecting the cap to the projection during the specimen collection process to hold the cap on the specimen collector and avoid introduction of the cap into the evidence collection scene.

## Claims

1. A method of avoiding contamination of an evidence collection scene the method comprising the steps of:
providing a specimen collector having a reagent holder thereon,
inserting a reagent vial into said reagent holder, said reagent vial having a cap thereon to close the vial,
providing a projection on the specimen collector the projection configured to hold the cap by either friction fit or by threaded connection,
removing the cap from the vial during specimen collection at a evidence collection scene, and
connecting the cap to the projection during the specimen collection process to hold the cap on the specimen collector and avoid introduction of the cap into the evidence collection scene.
